# EUROPEAN PATENT SPECIFICATION

(11) **EP 3 470 053 B1**
(45) Date of publication and mention of the grant of the patent: **09.12.2020**
(21) Application number: 17813526.5
(22) Date of filing: 09.06.2017
(51) Int. Cl.: A61K 8/891, A61K 8/894, A61K 8/29, A61K 8/27, A61K 8/37, A61K 8/06, A61Q 17/04, A61Q 19/00

(54) **ODORLESS AND HIGHLY STABLE COSMETIC COMPOSITION**
GERUCHLOSE UND HOCHSTABILE KOSMETISCHE ZUSAMMENSETZUNG
COMPOSITION COSMÉTIQUE INODORE ET TRÈS STABLE

(30) Priority: 13.06.2016 KR 20160073404
(43) Date of publication of application: 17.04.2019
(73) Proprietor: Amorepacific Corporation, Seoul 04386 (KR)
(72) Inventor: KANG, Shinbeom, Yongin-si Gyeonggi-do 17074 (KR); CHOI, Kyung Ho, Yongin-si Gyeonggi-do 17074 (KR); SHIN, Hong-Ju, Yongin-si Gyeonggi-do 17074 (KR); BAEK, Doo Hyun, Yongin-si Gyeonggi-do 17074 (KR)
(74) Representative: Novagraaf Technologies
(86) International application number: PCT/KR2017/006037
(87) International publication number: WO 2017/217702

(56) References cited:
- WO-A1-2013/100207
- WO-A2-2008/149279
- KR-A- 20120 042 397
- KR-A- 20120 049 078
- KR-A- 20120 139 465
- KR-A- 20120 139 465
- KR-A- 20150 011 887
- US-A1- 2003 108 498

## Description

### [Technical Field]

The present specification discloses a cosmetic composition having contents with improved odor and which has high stability.

### [Background Art]

In order to meet the need for safe UV-protection cosmetics, there have been developed UV-protection cosmetics which exclude an organic UV-blocking agent that may cause skin irritation, and which contain a large amount of inorganic UV-blocking powders of a high specific gravity.

However, the cosmetics give matt feeling due to the large amount of powders. Also, they have a problem that the amount of the cosmetic composition taken when the composition is applied is low because they have a low liquid content relative to the total weight thereof.

In addition, cosmetic compositions in the form of a W/O emulsion formulation containing a large amount of inorganic UV-blocking powders of a high specific gravity generally contain a large amount of a silicone acrylate dispersant, which has a high affinity to inorganic UV-blocking powders, in order to achieve stability. However, they have problems that the contents have a specific odor and the production cost per unit is high.

### [Citation List]

### [Patent Literature]

### [Patent Literature 1]

Korean Patent No. 1450387 and Korean Patent application KR20120139465 A 20121227.

### [Summary of Invention]

### [Technical Problem]

In one aspect, the object of the present invention is to provide a W/O type cosmetic composition exhibiting excellent stability without containing a silicone acrylate dispersant, which causes a specific odor. For all the following passages it is noted that the invention is strictly limited to the scope of the appended claims.

### [Technical Solution]

In order to achieve the above object, one embodiment of the present invention provides a cosmetic product comprising: a silicone acrylate-free, W/O type cosmetic composition comprising an inorganic UV-blocking agent and a silicone elastomer emulsifier; and a carrier comprising a sponge in which the cosmetic composition is impregnated, wherein the weight ratio of the inorganic UV-blocking agent and the silicone elastomer emulsifier is 10-30: 0.01-5 and the viscosity of the W/O type cosmetic composition is 5,000 to 35,000cps (centipoise).

### [Advantageous Effects]

The cosmetic composition according to one embodiment of the present invention comprises, as a dispersant, a silicone elastomer as an emulsifier in place of a silicone acrylate dispersant, which is used to allow to include a large amount of inorganic UV-blocking agents, which are powders of a high specific gravity, and thus can prevent the generation of a specific odor resulting from silicone acrylate, thus allowing even users sensitive to fragrance to use it without any feeling of discomfort. In addition, since the silicone elastomer has a network structure and thus stably traps an inorganic UV-blocking agent even in a low-viscosity cosmetic composition, the cosmetic composition has excellent formulation stability, so that, when the low-viscosity cosmetic composition is impregnated in a carrier comprising a sponge, the composition can have excellent filling, impregnating, and discharging abilities. Thus, the present invention can solve the problems of conventional low-viscosity cosmetic compositions that the liquid content is low due to the large amount of inorganic UV-blocking agents included therein and thus that the amount of the cosmetic composition taken from a foam carrier (discharged amount) is low.

### [Description of Drawings]

FIG. 1 is a diagram showing the action of an inorganic UV-blocking agent (pigment) as a dispersant in a W/O type composition of a silicone elastomer according to one embodiment of the present invention.
FIG. 2 is a diagram comparing formulation stability between an example of the present invention and comparative examples.
FIG. 3 is a diagram comparing formulation stability between an example of the present invention and comparative examples.
FIG. 4 is a diagram illustrating a method of comparing the amounts of cosmetic compositions discharged through an applicator in one embodiment of the present invention.
FIG. 5 is a diagram comparing the amounts of the cosmetic composition of an example of the present invention and of comparative examples discharged from a carrier.

### [Mode for Invention]

Hereinafter, preferred embodiments of the present invention will be described in detail so that those skilled in the art can easily carry out the present invention.

As used herein, the term "carrier" refers to a material capable of supporting any material, such as a composition, or any ingredient, and is used interchangeably with "support", "impregnation material" or "medium". A composition impregnated in the carrier may be delivered to the skin through an applicator (also referred to as an application means), including a hand, a puff, a tip, a brush, etc.

As used herein, the term "foam" refers to one formed by foaming a polymer through a dry or wet foaming process.

As used herein, the terms "impregnating capacity" and "impregnating ability" refer to the ability of holding and retaining any material or ingredient. The impregnating capacity required for a carrier is different from temporarily taking a material on an applicator, etc., in that the impregnating capacity relates to homogeneously supporting a composition for a long period of time.

As used herein, the terms "filling capacity" and "filling ability" refer to the ability of filling a cosmetic composition in a foam and may be expressed as the time required for filling a foam with a predetermined amount of a cosmetic composition. Herein, a measurement of "filling capacity" or "filling ability" refers to the time required for manually filling a foam having a diameter of 44mm and a height of 9.0mm with a cosmetic composition of 5,000 to 35,000cps.

As used herein, the terms "discharging capacity" and "discharging ability" refer to the amount of a cosmetic composition discharged when the cosmetic composition is taken from a foam in which the cosmetic composition is impregnated. It is preferable that a cosmetic composition be discharged from the foam in an adequate amount neither too little nor too much. Herein, a measurement of "discharging capacity" or "discharging ability" refers to the measurement obtained when compressing the surface of a foam having a diameter of 44mm and a height of 9.0mm and which is filled with a cosmetic composition of 5,000 to 35,000cps, with a load (force) of 2 kg.

As used herein, the terms "stability" and "formulation stability" refer to the degree at which a cosmetic composition impregnated in a foam maintains its formulation to the extent that its ingredients will not be separated from each other and will be maintained homogeneous when the foam is allowed to stand at a predetermined temperature for a predetermined period of time.

As used herein, the term "emulsifier" refers to a compound having emulsifying capacity and powder dispersing ability and is distinguished from "dispersant", which has only powder dispersing ability and no emulsifying capacity.

As used herein, the term "silicone elastomer emulsifier" is a subordinate concept of "silicone emulsifier". Among silicone emulsifiers, those having viscoelasticity, that is, both viscosity and elasticity, are classified as "silicone elastomer emulsifier".

One embodiment of the present invention provides a cosmetic product comprising: a W/O type cosmetic composition comprising an inorganic UV-blocking agent and a silicone elastomer emulsifier; and a carrier comprising a sponge in which the cosmetic composition is impregnated.

More specifically, one embodiment of the present invention provides a cosmetic product comprising: a silicone acrylate-free, W/O type cosmetic composition comprising an inorganic UV-blocking agent and a silicone elastomer emulsifier; and a carrier comprising a sponge in which the cosmetic composition is impregnated, wherein the weight ratio of the inorganic UV-blocking agent and the silicone elastomer emulsifier is 10-30: 0.01-5 and the viscosity of the W/O type cosmetic composition is 5,000 to 35,000cps (centipoise).

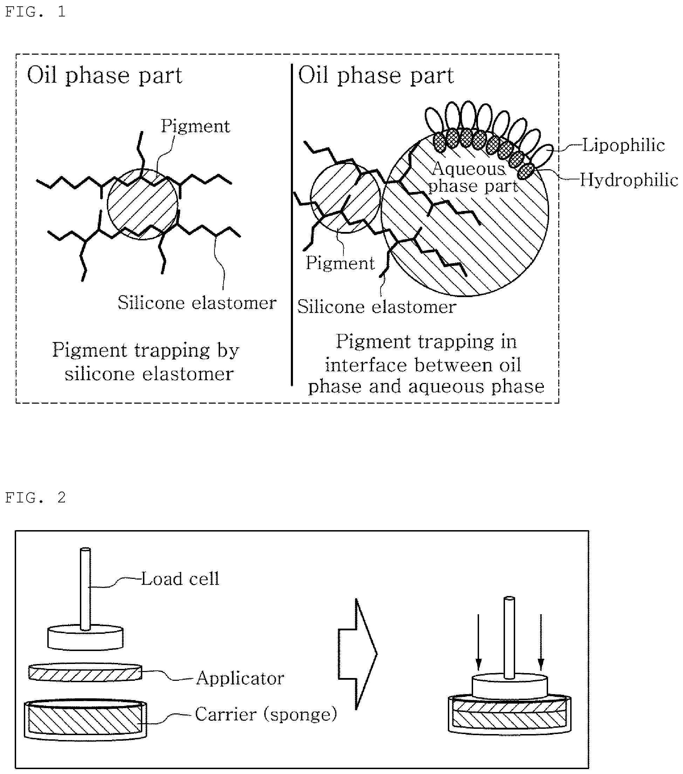

Hereinafter, the silicone elastomer emulsifier according to one embodiment will be described in more detail with reference to FIG. 1. As shown in FIG. 1, the silicone elastomer has a network structure and thus can trap pigments in the voids formed in the structure. Thus, when the silicone elastomer is included in a W/O type composition, it can stably trap pigments at the interface between the oil phase part (oil) and the aqueous phase part (water). Thus, in one embodiment of the present invention, the silicone elastomer as an emulsifier can be used as a dispersant of the inorganic UV-blocking agent, which is a powder of a high specific gravity, in a low-viscosity W/O type cosmetic composition, and can achieve excellent formulation stability. In addition, unlike conventional silicone acrylate dispersants, the silicone elastomer emulsifier has less or no specific odor and thus can improve the satisfaction of users who have a high sensitivity to odor. In one embodiment, the cosmetic composition is a silicone acrylate-free composition that does not comprise a silicone acrylate dispersant.

In one embodiment, the silicone elastomer emulsifier may comprise at least one selected from the group consisting of: dimethicone/PEG-10/15 crosspolymer, dimethicone/polyglycerin-3 crosspolymer, dimethicone/bis-isobutyl PPG-20 crosspolymer, dimethicone/PEG-10 crosspolymer, dimethicone/PEG-15 crosspolymer, dimethicone/PPG-20 crosspolymer, lauryl dimethicone PEG-15 crosspolymer, PEG-12 dimethicone/bis-isobutyl PPG-20 crosspolymer, PEG-10 dimethicone crosspolymer, PEG-12 dimethicone crosspolymer, PEG-8 dimethicone/polysorbate 20 crosspolymer, PEG-12 dimethicone/PPG-20 crosspolymer, PEG-10 dimethicone/vinyl dimethicone crosspolymer, PEG-10/lauryl dimethicone crosspolymer, PEG-15/lauryl dimethicone crosspolymer, PEG-15/lauryl polydimethylsiloxyethyl dimethicone crosspolymer, PEG-10 polydimethylsiloxyethyl dimethicone/bis-vinyl dimethicone crosspolymer, and polyglyceryl-3/lauryl polydimethylsiloxyethyl dimethicone crosspolymer, although not limited thereto.

The structures of the dimethicone/PEG-10/15 crosspolymer and the dimethicone/polyglycerin-3 crosspolymer in one embodiment are represented by the following formulae:

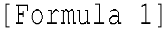

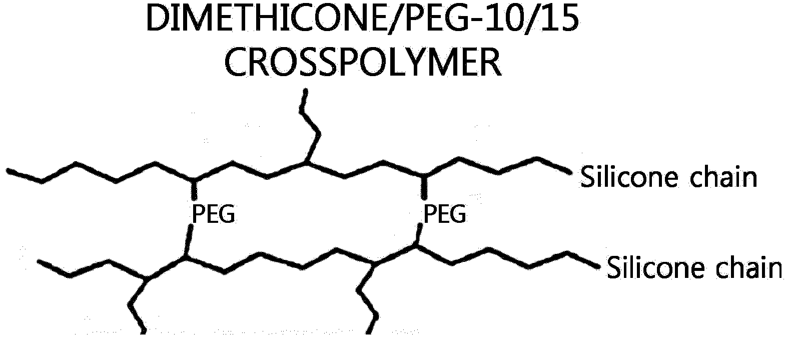

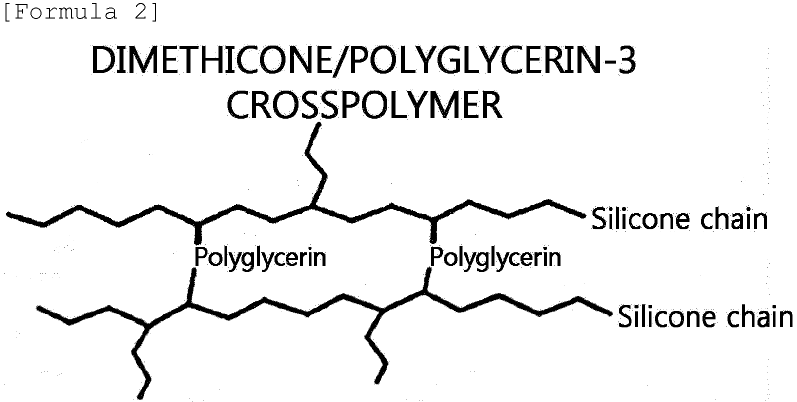

In one embodiment, the content of the silicone elastomer emulsifier may be more than 0, 0.01% by weight or more, 0.1% by weight or more, 1% by weight or more, 2% by weight or more, 3% by weight or more, 4% by weight or more or 5% by weight, or 5% by weight or less, 4% by weight or less, 3% by weight or less, 2% by weight or less, 1% by weight or less, or 0.1% by weight or less based on the total weight of the composition. Specifically, the content of the silicone elastomer emulsifier may be 0.01 to 5% by weight or 0.1 to 3% by weight, more specifically 1.5 to 2.5% by weight based on the total weight of the composition. If the content of the silicone elastomer emulsifier is less than 0.01% by weight, the inorganic UV-blocking agent is not uniformly dispersed in the formulation of a W/O type composition. If the content exceeds 5% by weight, it is difficult to stabilize the formulation.

In one embodiment, the W/O type cosmetic composition may further comprise a silicone emulsifier in addition to the silicone elastomer emulsifier. The silicone emulsifier may comprise, for example, at least one selected from the group consisting of: PEG-10 dimethicone, lauryl PEG-9 polydimethylsiloxyethyl dimethicone, acrylate/methoxy PEG-4 methacrylate copolymer, bis-cetyl/PEG-8 cetyl PEG-8 dimethicone, DEA PG-propyl PEG/PPG-18/21 dimethicone, lauryl PEG-8 dimethicone, lauryl PEG-9 polydimethylsiloxyethyl dimethicone, lauryl PEG/PPG-18/18 methicone, and lauryl PEG-10 tris(trimethylsiloxy)silylethyl dimethicone, although not limited thereto.

In one embodiment, the content of the silicone emulsifier may be 0.1% by weight or more, 1% by weight or more, 2% by weight or more, 3% by weight or more, 4% by weight or more, 5% by weight or more, 6% by weight or more, 7% by weight or more, 8% by weight or more, 9% by weight or more or 10% by weight or more, or 10% by weight or less, 9% by weight or less, 8% by weight or less, 7% by weight or less, 6% by weight or less, 5% by weight or less, 4% by weight or less, 3% by weight or less, 2% by weight or less, or 1% by weight or less.

In one embodiment, the inorganic UV-blocking agent may comprise at least one selected from the group consisting of microparticulate titanium dioxide and zinc oxide, although not limited thereto. The inorganic UV-blocking agent may be appropriately selected and used depending on the intended use such as UV barrier function, spreadability, and color. As the average particle size of the inorganic UV-blocking agent increases, the white turbidity occurring upon its application to the skin becomes more severe, thus decreasing the value of the product. On the other hand, if the particle size is too small, it may penetrate into the skin to cause irritation. Thus, in view of white turbidity, it is preferable that the average particle size of the inorganic UV-blocking agent be less than or equal to 300nm. In view of both skin irritation and white turbidity, it is preferable that the average particle size of the inorganic UV-blocking agent be 10-50nm.

In one embodiment, the content of the UV-blocking agent may be 0.1% by weight or more, 1% by weight or more, 3% by weight or more, 5% by weight or more, 7% by weight or more, 9% by weight or more, 11% by weight or more, 12% by weight or more, 14% by weight or more, 16% by weight or more, 18% by weight or more, 20% by weight or more, 22% by weight or more, 24% by weight or more, 26% by weight or more, 28% by weight or more, 30% by weight or more, 32% by weight or more, 34% by weight or more, 36% by weight or more, 38% by weight or more, or 40% by weight or more, or 40% by weight or less, 38% by weight or less, 36% by weight or less, 34% by weight or less, 32% by weight or less, 30% by weight or less, 28% by weight or less, 26% by weight or less, 24% by weight or less, 22% by weight or less, 20% by weight or less, 18% by weight or less, 16% by weight or less, 14% by weight or less, 12% by weight or less, 10% by weight or less, 9% by weight or less, 8% by weight or less, 7% by weight or less, 6% by weight or less, 5% by weight or less, 4% by weight or less, 3% by weight or less, 2% by weight or less or 1% by weight or less, based on the total weight of the composition. Specifically, the content of the UV-blocking agent may be 5 to 30% by weight or 10 to 25% by weight, more specifically 15 to 22% by weight, based on the total weight of the composition. If the content of the UV-blocking agent is less than 5% by weight, the UV blocking effect is insignificant. If the content exceeds 30% by weight, it is difficult to disperse it in a W/O type formulation.

In one embodiment, the W/O type cosmetic composition may comprise the inorganic UV-blocking agent and the silicone elastomer emulsifier at a weight ratio of 10-30: 0.01-5 in order to obtain the dispersion stability of the inorganic UV-blocking agent in the formulation. More specifically, the weight ratio may be 1: 0.01-0.5 or 1: 0.045-0.2.

In one embodiment, the W/O type cosmetic composition may further comprise an organic UV-blocking agent.

The organic UV-blocking agent may comprise, for example, at least one of ethylhexyl methoxycinnamate, octocrylene, avobenzene, butyl methoxydibenzoylmethane, oxybenzone, octyl triazone, menthyl anthranilate, 3,4-methylbenzylidene camphor and bis-ethylhexyloxyphenol methoxyphenyl triazine, although not limited thereto. The organic UV-blocking agent may be appropriately selected and used depending on the intended use such as UV barrier function, spreadability, and color.

In one embodiment, the content of the organic UV-blocking agent may be 0.1% by weight or more, 1% by weight or more, 2% by weight or more, 3% by weight or more, 4% by weight or more, 5% by weight or more, 6% by weight or more, 7% by weight or more, 8% by weight or more, 9% by weight or more, 10% by weight or more, 11% by weight or more, 12% by weight or more, 13% by weight or more, 14% by weight or more or 15% by weight or more, or 15% by weight or less, 14% by weight or less, 13% by weight or less, 12% by weight or less, 11% by weight or less, 10% by weight or less, 9% by weight or less, 8% by weight or less, 7% by weight or less, 6% by weight or less, 5% by weight or less, 4% by weight or less, 3% by weight or less, 2% by weight or less, 1% by weight or less or 0.1% by weight or less, based on the total weight of the composition. Specifically, the content of the organic UV-blocking agent may be 0.1 to 15% by weight or 0.1 to 10% by weight, more specifically 5 to 10% by weight based on the total weight of the composition. If the content of the organic UV-blocking agent is less than 0.1% by weight, the UV blocking effect is insignificant. If the content exceeds 10% by weight, it may cause irritation to the skin.

In one embodiment, the W/O type cosmetic composition of the present invention may comprise 40 to 80% by weight of oil phase part ingredients and 20 to 60% by weight of aqueous phase part ingredients, based on the total weight of the composition.

In one embodiment, the content of the oil phase part ingredients may be specifically 50 to 70% by weight or about 60% by weight, based on the total weight of the composition. The oil phase part ingredients may comprise the inorganic UV-blocking agent and the silicone elastomer emulsifier. In addition thereto, the oil phase part ingredients may further comprise an organic UV-blocking agent, a silicone oil such as cyclopentasiloxane or phenyl trimethicone, a thickener such as disteardimonium hectorite, a colorant, etc. The oil phase part ingredients may vary depending on the feeling of use and functionality of the formulation.

In one embodiment, the content of the aqueous phase part ingredients may be, more specifically, 30 to 50% by weight or about 40% by weight, based on the total weight of the composition. The aqueous phase part ingredients may comprise purified water, propanediol, a polyol such as butylene glycol or glycerin, sodium chloride, disodium EDTA, caprylyl glycol, etc., although not limited thereto. The aqueous phase part ingredients may vary depending on the feeling of use and functionality of the formulation.

In one embodiment of the present invention, the W/O type cosmetic composition may be a low-viscosity composition. The viscosity of the composition is not particularly limited, but may be 5,000 to 35,000cps (centipoise). Specifically, the viscosity of the cosmetic composition may be 50,000cps or lower, 40,000cps or lower, 35,000cps or lower, 30,000cps or lower, 20,000cps or lower, 10,000cps or lower, 7,000cps or lower or 5,000cps or lower and 5,000cps or higher, 7,000cps or higher, 8,000cps or higher, 10,000cps or higher, 20,000cps or higher or 35,000cps.

If the viscosity of the cosmetic composition is lower than 3,000cps, a low viscosity of the composition may cause deposition in the container or separation of the contents themselves even after impregnation. If the viscosity is higher than 30,000 cps, it may deteriorate the filling ability.

Herein, the viscosity may be measured using a viscosity measuring instrument, for example, LVDV II+PRO with spindle NO. 63 at a spindle speed of 5 RPM or 12 RPM, preferably with liner 3500-5000 and spindle No. 63 at 12 rpm. Alternatively, the viscosity may be measured using BROOKFIELD RVDV-III ULTRA (Serial No. RY6521152) with spindle 64 at 12 rpm. In the case of a liquid cosmetic composition, the viscosity may be measured using Brookfield DV-III ULTRA with spindle No. 63 at a spindle speed of 5 rpm.

Preferably, 5,000 to 35,000cps of the liquid cosmetic composition of may be measured at 35°C for 60 seconds, although not limited thereto.

The W/O type cosmetic composition according to one embodiment of the present invention may be used for UV blocking. For example, the UV-blocking composition may have a sun protection factor (SPF) of 30 or more. Also, the UV-blocking composition may have a protection grade of UVA (PA) of double plus (PA++) to triple plus (PA+++).

In addition, the W/O type cosmetic composition according to one embodiment may further comprise at least one of colorants, surfactants, moisturizers, thickeners, lubricants, preservatives, fragrances and other additives which are commonly used in the art, in addition to the above ingredients.

In one embodiment of the present invention, the formulation of the W/O type cosmetic composition is not particularly limited, but may be one selected from the group consisting of solutions, emulsions, gels, creams, and suspensions.

In one embodiment of the present invention, the W/O type cosmetic composition may be selected from the group consisting of cosmetic compositions for skin care, cosmetic compositions for make-up, cosmetic compositions for hair care, and sunscreens. The W/O type cosmetic composition may be formulated into, for example, a make-up primer, a make-up base, a liquid or solid foundation, a concealer, a lipstick, a lip gloss, a powder, a lip liner, an eye liner, a mascara, an eyebrow, an eyeshadow, a blusher, a twin cake, a sunscreen, a lotion, a cream, an essence or the like, although not limited thereto.

In one embodiment of the present invention, the W/O type cosmetic composition according to the present invention as described above is impregnated in a carrier comprising a sponge, thereby providing a cosmetic product with high stability and portability. In addition, the cosmetic product do not require hand washing after the cosmetic composition is taken from the carrier and applied to the skin, so that the user can conveniently enjoy lasting make-up effect or UV-blocking effect outdoors.

As used herein, the term "sponge" refers to a material capable of supporting any material, such as a composition, or any ingredient. It means rubbers, fibers or resins formed like a sea sponge and which can support and discharge a cosmetic composition. In one embodiment, the sponge is not particularly limited, but may be a natural sponge or a synthetic sponge. For example, the natural sponge may be a sea sponge, a natural rubber sponge or the like. The synthetic sponge may be a synthetic resin sponge, foamed urethane, latex, acrylonitrile-butadiene rubber (NBR), butadiene rubber (BR), styrene-butadiene rubber (SBR), natural rubber (NR), chloroprene rubber (CR), butyl rubber (isoprene-isobutylene rubber (IIR)), isoprene rubber (IR), vulcanized ethylene-propylene rubber (EPR), polysulfide rubber, silicone rubber, fluororubber, urethane rubber, acrylic rubber, ethylene propylene diene monomer (EPDM) rubber, polyvinyl alcohol (PVA), ethylene vinyl acetate (EVA), nitrile rubber (NR), or the like.

Herein, the foamed urethane, which is a type of sponge, is not particularly limited. However, in one embodiment, it may comprise polyether-based foamed urethane (containing ether polyol as the main base), polyester-based foamed urethane and polycarbonate-based foamed urethane. Specifically, the foamed urethane may comprise polyether-based foamed urethane.

In one embodiment, the polyether-based foamed urethane comprises polyether-based dry foamed urethane and polyether-based wet foamed urethane. The polyester-based foamed urethane comprises polyester-based dry foamed urethane and polyester-based wet foamed urethane. The polycarbonate-based foamed urethane comprises polycarbonate-based dry foamed urethane and polycarbonate-based wet foamed urethane.

In one embodiment, the sponge may include pores, so that the W/O type cosmetic composition according to one embodiment of the present invention can be impregnated in the pores of the sponge, thereby achieving uniform distribution in the carrier.

In one embodiment, the sponge may include pores of an average size of 30-2,500µm. The pore size is the average value of measurements obtained with an optical microscope (NIKON ECLIPSE 80i). If the average pore size of the sponge is less than 30µm, the pore space to hold a cosmetic composition is too small to provide good impregnating ability. If the average pore size exceeds 2500µm, the pore space is too large to provide good controllability in discharging of the composition. As used herein, the term "impregnating capacity" refers to the ability of holding and retaining any material or ingredient. The impregnating capacity required for a carrier is different from temporarily taking a material on an applicator, etc., in that the impregnating capacity relates to homogeneously supporting a composition for a long period of time. The terms "discharging capacity" and "discharging ability" refer to the amount of a cosmetic composition discharged when the cosmetic composition is taken from a foam in which the cosmetic composition is impregnated. It is preferable that a cosmetic composition be discharged in an adequate amount neither too little nor too much.

In one embodiment, the sponge may include 55-130 pores per inch (ppi). If the number of pores per inch of the sponge is greater than 130 ppi, the pore size is too small to control the fluidity of the cosmetic composition and the absorption or discharge of the cosmetic composition. If the number of pores per inch of the sponge is less than 55 ppi, the sponge may show poor impregnating ability after a cosmetic composition is impregnated. In one embodiment of the present invention, the term "the number of pores" refers to the number of pores per inch of a urethane foam. Herein, the number of pores may mean the average number of pores per inch of a horizontal or vertical line measured according to WI-QA-14 (ASTM).

In the present invention, the sponge has a density of 0.1 to 0.18 g/cm³. If the sponge has a density of less than 0.05 g/cm³, the cosmetic composition is discharged in an excessively large amount, resulting in inconvenience of use. When the sponge has a density of more than 0.2 g/cm³, the cosmetic composition may not be filled and discharged well.

In one embodiment of the present invention, the sponge may have a thickness of 1mm to 50mm. When the sponge has a thickness of less than 1mm, it cannot support a sufficient amount of a cosmetic composition. When the foam has a thickness of more than 50mm, it is difficult to discharge a cosmetic composition without residue of the contents. For example, the carrier using the sponge according to one embodiment of the present invention may have a thickness of 3mm to 45mm, for example, 5mm to 40mm, for example, 8mm to 35mm, for example, 10mm to 30mm. Specifically, the carrier may have a thickness of 50mm or less, 40mm or less, 30mm or less, 20mm or less, 10mm or less, or 5mm or less and 1mm or more, 10mm or more, 20mm or more, 30mm or more, 40mm or more, or 50mm or more.

In addition, in the cosmetic product according to one embodiment of the present invention, the W/O type cosmetic composition is impregnated in a carrier comprising the sponge, so that the cosmetic composition is filled well therein and the cosmetic composition can be homogeneously supported for a long period of time. Also, when the cosmetic composition is taken, it can be discharged in an appropriate amount. Further, the cosmetic composition can maintain excellent formulation stability for a long period of time.

The cosmetic product according to one embodiment of the present invention may be provided in a cosmetic container generally called "compact", which comprises a lower part capable of accommodating a carrier for a cosmetic composition and an upper lid part to which a mirror, etc. may be attached. The "compact" may further comprise, in addition to a carrier, an applicator for taking the composition impregnated in the carrier and applying it to the skin.

In addition, the cosmetic product according to one embodiment of the present invention may further comprise an applicator for taking the cosmetic composition from the sponge carrier in which the W/O type cosmetic composition is impregnated. Further, the cosmetic product may further comprise a cosmetic container generally called "compact", which comprises a lower part capable of accommodating a carrier for a cosmetic composition, a mirror, etc., and an upper lid part, although not limited thereto.

Hereinafter, the present invention will be described in detail by way of examples.

### [Preparation Example]

W/O type cosmetic compositions were prepared by a conventional method in the art with the composition shown in the following Table 1. Specifically, the following organic UV-blocking agent, oil phase part ingredients, dispersant, and colorant were mixed and stirred at room temperature to a homogeneous state, and then the resultant mixture was further mixed with the following thickener and stirred to a homogeneous state. Then, an inorganic UV-blocking agent was added and the resultant mixture was stirred to a homogeneous state. In a separate mixer, the following aqueous phase part ingredients were mixed and stirred at room temperature so that they were dissolved completely. The uniformly stirred aqueous phase part ingredients were gradually introduced to the prepared mixture containing oil phase part ingredients and the mixture was emulsified with a homogenizer. The resultant was cooled to obtain each of Examples 1 and 2 and Comparative Examples 1 to 6. The viscosity of each of the thus-obtained compositions on the next day after preparation was as follows: Example 1=12,500cps, Example 2=12,000cps, Comparative Example 1=16,500cps, Comparative Example 2=15,700cps, Comparative Example 3=16,000cps, Comparative Example 4=17,000cps, Comparative Example 5=16,200cps, and Comparative Example 6=18,300cps. (cps= centipoises = plural of centipoise = 1 cP = 10-3 Pa·s = 1 mPa·S (SI-units))

**[Table 1]**

| Ingredients | | Content (% by weight relative to the total weight of the composition) | | | | | | | |
|---|---|---|---|---|---|---|---|---|---|
| | | Organic+inorganic UV-blocking agents | | | | Inorganic UV-blocking agent | | | |
| | | Comparative Example 1 | Comparative Example 2 | Comparative Example 3 | Example 1 | Comparative Example 4 | Comparative Example 5 | Comparative Example 6 | Example 2 |
| Organic UV-blocking agent | Ethylhexyl methoxycinnamate | 7.00 | 7.00 | 7.00 | 7.00 | 0.00 | 0.00 | 0.00 | 0.00 |
| Oil phase part ingredients | Cyclopentasiloxan e | 16.7 5 | 18.7 5 | 17.7 5 | 23.7 5 | 16.7 5 | 18.7 5 | 17.7 5 | 23.2 5 |
| | Phenyl trimethicone | 6.00 | 6.00 | 6.00 | 6.00 | 6.00 | 6.00 | 6.00 | 6.00 |
| Dispersant/emul sifier | Acrylate/ethylhex yl acrylate/dimethic one methacrylate copolymer | 3.00 | 3.00 | 3.00 | 0.00 | 3.00 | 3.00 | 3.00 | 0.00 |
| | Polyhydroxysteari c acid | 2.00 | 2.00 | 2.00 | 0.00 | 2.00 | 2.00 | 2.00 | 0.00 |
| | Lauryl PEG-9 polydimethylsilox yethyl dimethicone | 4.00 | 4.00 | 2.00 | 2.00 | 4.00 | 4.00 | 2.00 | 2.50 |
| | PEG-10 dimethicone | 4.00 | 4.00 | 5.00 | 4.00 | 4.00 | 4.00 | 5.00 | 4.00 |
| | Dimethicone/PEG-10/15 crosspolymer | 2.00 | 0.00 | 2.00 | 2.00 | 2.00 | 0.00 | 2.00 | 2.00 |
| Thickener | Disteardimonium hectorite | 0.20 | 0.20 | 0.20 | 0.20 | 0.20 | 0.20 | 0.20 | 0.20 |
| Inorganic UV-blocking agent | Microparticulate titanium dioxide | 5.00 | 5.00 | 5.00 | 5.00 | 10.0 0 | 10.0 0 | 10.0 0 | 10.0 0 |
| | Zinc oxide | 10.0 0 | 10.0 0 | 10.0 0 | 10.0 0 | 12.0 0 | 12.0 0 | 12.0 0 | 12.0 0 |
| Colorant | Iron oxide | 1.50 | 1.50 | 1.50 | 1.50 | 1.50 | 1.50 | 1.50 | 1.50 |
| Aqueous phase part ingredients | Purified water | 34.0 0 | 34.0 0 | 34.0 0 | 34.0 0 | 34.0 0 | 34.0 0 | 34.0 0 | 34.0 0 |
| | Propanediol | 3.50 | 3.50 | 3.50 | 3.50 | 3.50 | 3.50 | 3.50 | 3.50 |
| | Sodium chloride | 0.80 | 0.80 | 0.80 | 0.80 | 0.80 | 0.80 | 0.80 | 0.80 |
| | Disodium EDTA | 0.05 | 0.05 | 0.05 | 0.05 | 0.05 | 0.05 | 0.05 | 0.05 |
| | Caprylyl glycol | 0.20 | 0.20 | 0.20 | 0.20 | 0.20 | 0.20 | 0.20 | 0.20 |
| | Total | 100. 00 | 100. 00 | 100. 00 | 100. 00 | 100. 00 | 100. 00 | 100. 00 | 100. 00 |

### [Test Example 1] Evaluation of formulation stability

25 ml of each of the cosmetic compositions of Examples 1 and 2 and Comparative Examples 1 to 4 were put in a 100 ml sample container. Then, the compositions were stirred at a rate of 185rpm while repeating a cycle of freezing and thawing in a temperature range of -10°C to 45.4°C for one week using a cycle chamber, whose temperature changes over time within a temperature range of -10°C to 45.4°C, to visually evaluate the formulation stability under extreme conditions. The formulation stability was determined by examining whether the formulation was separated or whether a band was visible that occurs when a pigment is separated.

**[Table 2]**

| | Comparative Example 1 | Comparative Example 2 | Comparative Example 3 | Example 1 |
|---|---|---|---|---|
| Image observed after 1 week of cycle shaking | Separation of inorganic UV-blocking agent on the wall | Separation of colorant and inorganic UV-blocking agent on the wall | Separation of colorant and inorganic UV-blocking agent on the wall and the upper part | Excellent stability |

**[Table 3]**

| | Comparative Example 4 | Comparative Example 5 | Comparative Example 6 | Example 2 |
|---|---|---|---|---|
| Image observed after 1 week of cycle shaking | Separation of inorganic UV-blocking agent on the wall | Separation of colorant and inorganic UV-blocking agent on the wall | Separation of colorant and inorganic UV-blocking agent on the wall and the upper part | Excellent stability |

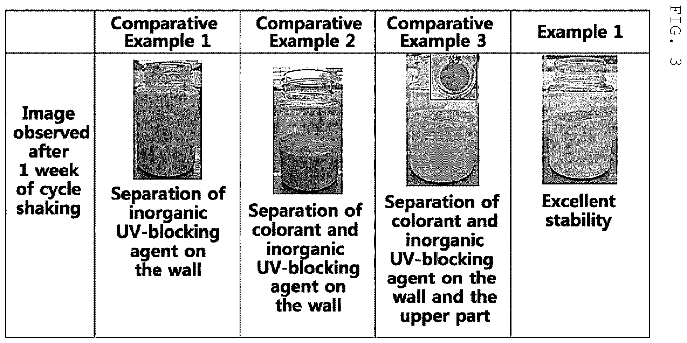

As a result, it was found that Examples 1 and 2, which comprised a silicone elastomer emulsifier instead of a silicone acrylate dispersant, achieved better formulation stability with less amount than Comparative Examples 1 to 6, which comprised a silicone acrylate, as shown in Tables 2 and 3 and FIGs. 2 and 3, which show the photographs thereof.

### [Test Example 2] Evaluation of discharging ability

The cosmetic compositions prepared in Example 2 and Comparative Example 5 were filled in a carrier comprising a sponge, and then their discharging ability during use was evaluated.

First, a urethane foam having a diameter of 44mm and a height of 9.0mm was put into a container, and 17g of each of the cosmetic compositions was manually filled therein using a spatula.

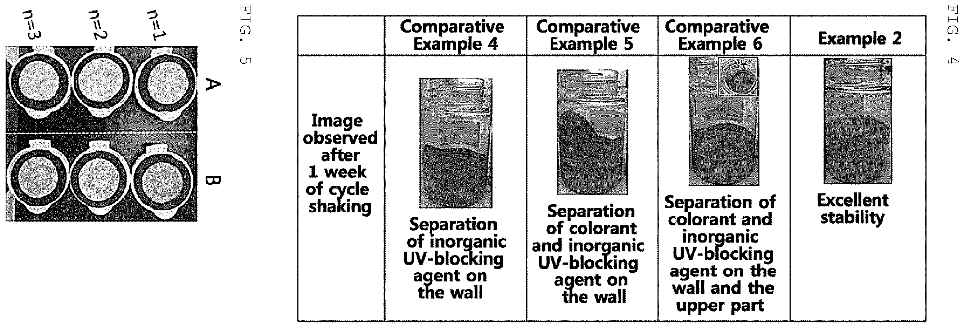

Next, the amount picked up by an applicator when a uniform force was applied by an apparatus was evaluated, by using a hardness measuring instrument having the structure of FIG. 4, in order to measure the discharged amount. The measurement conditions were as follows, and the same procedure was repeated three times.
- Entry depth into the composition: 10mm
- Head speed of the load cell: 20 mm/min
- Φ = 25mm
- Retention time (t) after entry of the load cell = lmin
- Maximum load of the load cell: 2kg

**[Table 4]**

| Comparative Example 5 | | | Example 2 | | |
|---|---|---|---|---|---|
| ① Applicator (g) | ② Weight of applicator+cos metic composition (g) | Cosmetic compositio n(g) (②-①) | ① Applicator (g) | ② Weight of applicator+cosm etic composition (g) | Cosmetic compositio n (g) (②-①) |
| 1.38 | 2.20 | 0.82 | 1.56 | 2.48 | 0.92 |
| 1.58 | 2.54 | 0.96 | 1.48 | 2.50 | 1.02 |
| 1.39 | 2.20 | 0.81 | 1.54 | 2.44 | 0.90 |
| Average (g) | | 0.86 | Average (g) | | 0.95 |

As a result, it was found that the discharged amount of Example 2 increased as compared to Comparative Example 5, due to an increase of the fluidity of the cosmetic product, as shown in FIG. 5. In addition, as shown in Table 4, which analyzes the discharged amounts, it was found that the discharged amount of the present invention increased by 10.47% compared to the comparative example, since the present invention, which includes a silicone elastomer instead of silicone acrylate, achieved a low-viscosity cosmetic composition with high formulation stability, resulting in increased fluidity of the fluid.

## Claims

1. A cosmetic product comprising: a silicone acrylate-free, W/O type cosmetic composition comprising an inorganic UV-blocking agent and a silicone elastomer emulsifier; and a carrier comprising a sponge in which the cosmetic composition is impregnated, wherein the weight ratio of the inorganic UV-blocking agent and the silicone elastomer emulsifier is 10-30 : 0.01-5, wherein the viscosity of the W/O type cosmetic composition is 5 to 35 Pa.s (5,000 to 35,000 centipoises), and wherein the sponge has a density of 0.1 to 0.18 g/cm³.

2. The cosmetic product according to claim 1, wherein the silicone elastomer emulsifier comprises at least one selected from the group consisting of: dimethicone/PEG-10/15 crosspolymer, dimethicone/polyglycerin-3 crosspolymer, dimethicone/bis-isobutyl PPG-20 crosspolymer, dimethicone/PEG-10 crosspolymer, dimethicone/PEG-15 crosspolymer, dimethicone/PPG-20 crosspolymer, lauryl dimethicone PEG-15 crosspolymer, PEG-12 dimethicone/bis-isobutyl PPG-20 crosspolymer, PEG-10 dimethicone crosspolymer, PEG-12 dimethicone crosspolymer, PEG-8 dimethicone/polysorbate 20 crosspolymer, PEG-12 dimethicone/PPG-20 crosspolymer, PEG-10 dimethicone/vinyl dimethicone crosspolymer, PEG-10/lauryl dimethicone crosspolymer, PEG-15/lauryl dimethicone crosspolymer, PEG-15/lauryl polydimethylsiloxyethyl dimethicone crosspolymer, PEG-10 polydimethylsiloxyethyl dimethicone/bis-vinyl dimethicone crosspolymer, and polyglyceryl-3/lauryl polydimethylsiloxyethyl dimethicone crosspolymer.

3. The cosmetic product according to claim 1, wherein the silicone elastomer emulsifier has a network structure.

4. The cosmetic product according to claim 1, wherein the W/O type cosmetic composition further comprises a silicone emulsifier.

5. The cosmetic product according to claim 4, wherein the silicone emulsifier comprises at least one selected from the group consisting of: PEG-10 dimethicone, lauryl PEG-9 polydimethylsiloxyethyl dimethicone, acrylate/methoxy PEG-4 methacrylate copolymer, bis-cetyl/PEG-8 cetyl PEG-8 dimethicone, DEA PG-propyl PEG/PPG-18/21 dimethicone, lauryl PEG-8 dimethicone, lauryl PEG-9 polydimethylsiloxyethyl dimethicone, lauryl PEG/PPG-18/18 methicone, and lauryl PEG-10 tris(trimethylsiloxy)silylethyl dimethicone.

6. The cosmetic product according to claim 1, wherein the inorganic UV-blocking agent comprises at least one selected from the group consisting of microparticulate titanium dioxide and zinc oxide.

7. The cosmetic product according to claim 1, wherein the W/O type cosmetic composition comprises 15 to 22% by weight of the inorganic UV-blocking agent and 0.1 to 3% by weight of the silicone elastomer emulsifier based on the total weight of the composition.

8. The cosmetic product according to claim 1, wherein the W/O type cosmetic composition further comprises an organic UV-blocking agent.

9. The cosmetic product according to claim 8, wherein the organic UV-blocking agent comprises at least one of octyl methoxycinnamate, ethylhexyl methoxycinnamate, octocrylene, avobenzene, butyl methoxydibenzoylmethane, oxybenzone, octyl triazone, menthyl anthranilate, 3,4-methylbenzylidene camphor, and bis-ethylhexyloxyphenol methoxyphenyl triazine.

10. The cosmetic product according to claim 8, wherein the W/O type cosmetic composition further comprises 0.1 to 10% by weight of the organic UV-blocking agent based on the total weight of the composition.

11. The cosmetic product according to claim 1, wherein the W/O type cosmetic composition has a sun protection factor (SPF) of 30 or more and a protection grade of UVA (PA) of double plus (PA++) to triple plus (PA+++).

12. The cosmetic product according to claim 1, wherein the W/O type cosmetic composition is selected from the group consisting of solutions, emulsions, gels, creams, and suspensions.

13. The cosmetic product according to claim 1, wherein the sponge comprises at least one selected from the group consisting of natural rubber, synthetic resin, foamed urethane, latex, acrylonitrile-butadiene rubber (NBR), butadiene rubber (BR), styrene-butadiene rubber (SBR), natural rubber (NR), chloroprene rubber (CR), butyl rubber (isoprene-isobutylene rubber (IIR)), isoprene rubber (IR), vulcanized ethylene-propylene rubber (EPR), polysulfide rubber, silicone rubber, fluororubber, urethane rubber, acrylic rubber, ethylene propylene diene monomer (EPDM) rubber, polyvinyl alcohol (PVA), ethylene vinyl acetate (EVA), and nitrile rubber (NR).

14. The cosmetic product according to claim 1, further comprising an applicator for taking the cosmetic composition from the carrier in which the W/O type cosmetic composition is impregnated.

## Patentansprüche

1. Kosmetisches Produkt, umfassend: eine silikonacrylatfreie kosmetische Zusammensetzung vom W/O-Typ, umfassend ein anorganisches UV-Blockierungsmittel und einen Silikonelastomer-Emulgator; und einen Träger, umfassend einen Schwamm, in dem die kosmetische Zusammensetzung imprägniert ist, wobei das Gewichtsverhältnis des anorganischen UV-Blockierungsmittels und des Silikonelastomer-Emulgators 10-30 : 0,01-5 beträgt, wobei die Viskosität der kosmetischen Zusammensetzung vom W/O-Typ 5 bis 35 Pa.s (5.000 bis 35.000 Centipoise) beträgt, und wobei der Schwamm eine Dichte von 0,1 bis 0,18 g/cm3 aufweist.

2. Kosmetisches Produkt nach Anspruch 1, wobei der Siliconelastomer-Emulgator mindestens einen umfasst, der aus der Gruppe ausgewählt ist, bestehend aus: Dimethicon/PEG-10/15-Kreuzpolymer, Dimethicon/Polyglycerin-3-Kreuzpolymer, Dimethicon/Bis-isobutyl-PPG-20-Kreuzpolymer, Dimethicon/PEG-10-Kreuzpolymer, Dimethicon/PEG-15-Kreuzpolymer, Dimethicon/PG-20-Kreuzpolymer, Lauryldimethicon-PEG-15-Kreuzpolymer, PEG-12-Dimethicon/Bisisisobutyl-PPG-20-Kreuzpolymer, PEG-10-Dimethicon-Kreuzpolymer, PEG-12-Dimethicon-Kreuzpolymer, PEG-8-Dimethicon/Polysorbat-20-Kreuzpolymer, PEG-12-Dimethicon/PPG-20-Kreuzpolymer, PEG-10-Dimethicon/Vinyl-Dimethicon-Kreuzpolymer, PEG-10/Lauryl-Dimethicon-Kreuzpolymer, PEG-15/Lauryl-Dimethicon-Kreuzpolymer, PEG-15/Lauryl-Polydimethylsiloxyethyl-Dimethicon-Kreuzpolymer, PEG-10-Polydimethylsiloxyethyl-Dimethicon/Bis-Vinyl-Dimethicon-Kreuzpolymer und Polyglyceryl-3/Lauryl-Polydimethylsiloxyethyl-Dimethicon-Kreuzpolymer.

3. Kosmetisches Produkt nach Anspruch 1, wobei der Silikonelastomer-Emulgator eine Netzwerkstruktur aufweist.

4. Kosmetisches Produkt nach Anspruch 1, wobei die kosmetische Zusammensetzung vom W/O-Typ weiterhin einen Silikon-Emulgator umfasst.

5. Kosmetisches Produkt nach Anspruch 4, wobei der Siliconemulgator mindestens einen Emulgator umfasst, ausgewählt aus der Gruppe bestehend aus: PEG-10-Dimethicon, Lauryl-PEG-9-Polydimethylsiloxyethyldimethicon, Acrylat/Methoxy-PEG-4-Methacrylat-Copolymer, Bis-Cetyl/PEG-8-Cetyl-PEG-8-Dimethicon, DEA PG-Propyl-PEG/PPG-18/21-Dimethicon, Lauryl PEG-8 Dimethicon, Lauryl PEG-9 Polydimethylsiloxyethyldimethicon, Lauryl PEG/PPG-18/18 Methicon und Lauryl PEG-10 Tris(trimethylsiloxy)silylethyldimethicon.

6. Kosmetisches Produkt nach Anspruch 1, wobei das anorganische UV-Blockierungsmittel mindestens ein Mittel umfasst, das aus der Gruppe ausgewählt ist, die aus mikroteilchenförmigem Titandioxid und Zinkoxid besteht.

7. Kosmetisches Produkt nach Anspruch 1, wobei die kosmetische Zusammensetzung vom W/O-Typ 15 bis 22 Gew.-% des anorganischen UV-Blockierungsmittels und 0,1 bis 3 Gew.-% des Silikonelastomer-Emulgators, bezogen auf das Gesamtgewicht der Zusammensetzung, umfasst.

8. Kosmetisches Produkt nach Anspruch 1, wobei die kosmetische Zusammensetzung vom W/O-Typ weiterhin ein organisches UV-Blockierungsmittel umfasst.

9. Kosmetisches Produkt nach Anspruch 8, wobei das organische UV-Blockierungsmittel mindestens eines der folgenden Mittel enthält: Octylmethoxycinnamat, Ethylhexylmethoxycinnamat, Octocrylen, Avobenzol, Butylmethoxydibenzoylmethan, Oxybenzon, Octyltriazon, Menthylanthranilat, 3,4-Methylbenzylidencampher und Bisethylhexyloxyphenolmethoxyphenyltriazin.

10. Kosmetisches Produkt nach Anspruch 8, wobei die kosmetische Zusammensetzung vom W/O-Typ weiterhin 0,1 bis 10 Gew.-% des organischen UV-Blockierungsmittels, bezogen auf das Gesamtgewicht der Zusammensetzung, enthält.

11. Kosmetisches Produkt nach Anspruch 1, wobei die kosmetische Zusammensetzung vom W/O-Typ einen Lichtschutzfaktor (SPF) von 30 oder mehr und einen Schutzgrad von UVA (PA) von doppelt plus (PA++) bis dreifach plus (PA+++) aufweist.

12. Kosmetisches Produkt nach Anspruch 1, wobei die kosmetische Zusammensetzung vom W/O-Typ ausgewählt ist aus der Gruppe bestehend aus Lösungen, Emulsionen, Gelen, Cremes und Suspensionen.

13. Kosmetisches Produkt nach Anspruch 1, wobei der Schwamm mindestens einen Schwamm umfasst, der aus der Gruppe ausgewählt ist, die aus Naturkautschuk, Kunstharz, geschäumtem Urethan, Latex, AcrylnitrilButadien-Kautschuk (NBR), Butadien-Kautschuk (BR), Styrol-Butadien-Kautschuk (SBR), Naturkautschuk (NR), Chloropren-Kautschuk (CR) besteht, Butylkautschuk (Isopren-Isobutylenkautschuk (IIR)), Isoprenkautschuk (IR), vulkanisierter Ethylen-Propylen-Kautschuk (EPR), Polysulfidkautschuk, Silikonkautschuk, Fluorkautschuk, Urethankautschuk, Acrylkautschuk, Ethylen-Propylen-Dien-Monomer-Kautschuk (EPDM), Polyvinylalkohol (PVA), EthylenVinylacetat (EVA) und Nitrilkautschuk (NR).

14. Kosmetisches Produkt nach Anspruch 1, das ferner einen Applikator zur Entnahme der kosmetischen Zusammensetzung von dem Träger, in dem die kosmetische Zusammensetzung vom W/O-Typ imprägniert ist, umfasst.

## Revendications

1. Produit cosmétique comprenant : une composition cosmétique de type E/H sans acrylate de silicone comprenant un agent inorganique de blocage des UV et un émulsifiant d'élastomère de silicone ; et un support comprenant une éponge dans laquelle la composition cosmétique est imprégnée, dans lequel le rapport en poids de l'agent inorganique de blocage des UV et de l'émulsifiant d'élastomère de silicone est de 10-30 : 0,01-5, dans lequel la viscosité de la composition cosmétique de type E/H est de 5 à 35 Pa.s (5 000 à 35 000 centipoises), et dans lequel l'éponge a une densité de 0,1 à 0,18 g/cm3.

2. Produit cosmétique selon la revendication 1, dans lequel l'émulsifiant élastomère de silicone comprend au moins un élément choisi dans le groupe constitué par : polymère croisé diméthicone/PEG-10/15, polymère croisé diméthicone/polyglycérine-3, polymère croisé diméthicone/bis-isobutyl PPG-20, polymère croisé diméthicone/PEG-10, polymère croisé diméthicone/PEG-15, polymère croisé diméthicone/PPG-20, polymère croisé lauryl diméthicone PEG-15, polymère croisé PEG-12 diméthicone/bis-isobutyl PPG-20, polymère croisé PEG-10 diméthicone, polymère croisé PEG-12 diméthicone, polymère croisé PEG-8 diméthicone/polysorbate 20, Polymère croisé PEG-12 diméthicone/PPG-20, Polymère croisé PEG-10 diméthicone/vinyl diméthicone, Polymère croisé PEG-10/lauryl diméthicone, Polymère croisé PEG-15/lauryl diméthicone, PEG-15/lauryl polydiméthylsiloxyéthyl diméthicone, PEG-10 polydiméthylsiloxyéthyl diméthicone / bis-vinyl diméthicone, et polyglycéryl-3/lauryl polydiméthylsiloxyéthyl diméthicone.

3. Produit cosmétique selon la revendication 1, dans lequel l'émulsifiant élastomère de silicone a une structure en réseau.

4. Produit cosmétique selon la revendication 1, dans lequel la composition cosmétique de type E/H comprend en outre un émulsifiant à base de silicone.

5. Produit cosmétique selon la revendication 4, dans lequel l'émulsifiant de silicone comprend au moins un élément choisi dans le groupe constitué par : PEG-10 diméthicone, lauryl PEG-9 polydiméthylsiloxyéthyl diméthicone, copolymère acrylate/méthoxy PEG-4 méthacrylate, bis-cétyl/PEG-8 cétyl PEG-8 diméthicone, DEA PG-propyl PEG/PPG-18/21 diméthicone, lauryl PEG-8 diméthicone, lauryl PEG-9 polydiméthylsiloxyéthyl diméthicone, lauryl PEG/PPG-18/18 méthicone, et lauryl PEG-10 tris(triméthylsiloxy)silyléthyl diméthicone.

6. Produit cosmétique selon la revendication 1, dans lequel l'agent inorganique de blocage des UV comprend au moins un élément choisi dans le groupe constitué par le dioxyde de titane microparticulaire et l'oxyde de zinc.

7. Produit cosmétique selon la revendication 1, dans lequel la composition cosmétique de type E/H comprend 15 à 22 % en poids de l'agent inorganique de blocage des UV et 0,1 à 3 % en poids de l'émulsifiant élastomère de silicone par rapport au poids total de la composition.

8. Produit cosmétique selon la revendication 1, dans lequel la composition cosmétique de type E/H comprend en outre un agent organique de blocage des UV.

9. Produit cosmétique selon la revendication 8, dans lequel l'agent organique de blocage des UV comprend au moins l'un des éléments suivants : méthoxycinnamate d'octyle, méthoxycinnamate d'éthylhexyle, octocrylène, avobenzène, butylméthoxydibenzoylméthane, oxybenzone, octyltriazone, anthranilate de menthyle, 3,4-méthylbenzylidène camphre et bis-éthylhexyloxyphénol méthoxyphényltriazine.

10. Produit cosmétique selon la revendication 8, dans lequel la composition cosmétique de type E/H comprend en outre 0,1 à 10 % en poids de l'agent organique de blocage des UV par rapport au poids total de la composition.

11. Produit cosmétique selon la revendication 1, dans lequel la composition cosmétique de type E/H a un facteur de protection solaire (SPF) de 30 ou plus et un degré de protection contre les UVA (PA) de double plus (PA++) à triple plus (PA+++).

12. Produit cosmétique selon la revendication 1, dans lequel la composition cosmétique de type E/H est choisie dans le groupe constitué par les solutions, les émulsions, les gels, les crèmes et les suspensions.

13. Produit cosmétique selon la revendication 1, dans lequel l'éponge comprend au moins un élément choisi dans le groupe constitué par le caoutchouc naturel, la résine synthétique, la mousse d'uréthane, le latex, le caoutchouc acrylonitrile-butadiène (NBR), le caoutchouc butadiène (BR), le caoutchouc styrène-butadiène (SBR), le caoutchouc naturel (NR), le caoutchouc chloroprène (CR), caoutchouc butyle (caoutchouc isoprène-isobutylène (IIR)), caoutchouc isoprène (IR), caoutchouc éthylène-propylène vulcanisé (EPR), caoutchouc polysulfure, caoutchouc silicone, caoutchouc fluoré, caoutchouc uréthane, caoutchouc acrylique, caoutchouc éthylène-propylène-diène monomère (EPDM), alcool polyvinylique (PVA), éthylène-acétate de vinyle (EVA) et caoutchouc nitrile (NR).

14. Produit cosmétique selon la revendication 1, comprenant en outre un applicateur pour prélever la composition cosmétique du support dans lequel la composition cosmétique de type E/H est imprégnée.
